# EUROPÄISCHE PATENTSCHRIFT

(11) **EP 0 570 769 B1**
(45) Veröffentlichungstag und Bekanntmachung des Hinweises auf die Patenterteilung: **20.03.1996**
(21) Anmeldenummer: 93107309.2
(22) Anmeldetag: 05.05.1993
(51) Int. Cl.: C07C 201/08, C07C 201/16, C07C 205/06, C07C 205/12

(54) **Verfahren zur Herstellung und Reinigung von Nitroaromaten**
Process for the preparation and purification of nitroaromatics
Procédé pour la préparation et la purification de composés nitroaromatiques

(30) Priorität: 18.05.1992 DE 4216416
(43) Veröffentlichungstag der Anmeldung: 24.11.1993
(73) Patentinhaber: BAYER AG, D-51368 Leverkusen (DE)
(72) Erfinder: Grenner, Dieter, Dr., W-5090 Leverkusen 1 (DE); Schieb, Thomas, Dr., W-5064 Rösrath (DE); Wiechers, Gerhard, Dr., W-5090 Leverkusen 3 (DE)

(56) Entgegenhaltungen:
- EP-A- 0 022 241
- US-A- 5 057 632

## Beschreibung

Die Erfindung betrifft ein Verfahren zur Herstellung und Reinigung von Nitroaromaten durch Nitrierung der entsprechenden Aromaten und anschließender Schmelzkristallisation mit Rückführung der anfallenden Restschmelze.

Nitroaromaten sind interessante Zwischenprodukte für die Synthese von Farbstoffen, Kunststoffvorprodukten, wie z.B. Toluylendiisocyanat und Diphenylmethandiisocyanat, Pflanzenschutzmitteln etc.. Die Nitroverbindungen werden im allgemeinen zu den entsprechenden Aminen reduziert, die dann weiterverarbeitet werden.

Nitroaromaten werden technisch durch Umsetzung der aromatischen Kohlenwasserstoffe mit Salpetersäure in flüssiger Phase in Gegenwart einer Lewis-Säure als Katalysator hergestellt. Speziell Großprodukte, wie Nitrobenzole und Nitrotoluole mit einer oder mehreren Nitrogruppen werden so gewonnen. Für die Umsetzung werden Mischungen aus Schwefelsäure und Salpetersäure wechselnder Konzentrationen eingesetzt. Daneben werden in der Literatur Verfahren beschrieben, die ausschließlich mit Salpetersäure (US 4 918 250, US 5 001 272, WO 89/12620) oder N₂O₄/O₂-Mischungen (EP 169 441 und EP 173 131) in flüssiger Phase arbeiten.

Allen Verfahren ist gemeinsam, daß eine Abtrennung der Nitroaromaten von der wäßrigen Säure und dem Reaktionswasser sowie eine Neutralisation der im Nitroaromaten gelösten Säuren mit basischen Substanzen (z.B. mit Metalloxiden, -hydroxiden, -carbonaten, -hydrogencarbonaten, Ammoniak, Aminen etc.) erfolgt. Diese Basen werden in Form von wäßrigen Lösungen eingesetzt, um den Nitroaromaten säurefrei zu waschen. Anschließend erfolgt noch eine Wäsche mit vollentsalztem Wasser, um gelöste Salze aus dem Nitroaromaten zu entfernen. Hierdurch entstehen größere Mengen Abwässer (ca. 50 bis 1000 kg Abwässer pro t Nitroaromaten), die stark mit anorganischen Salzen, wie Nitraten und Sulfaten, und nitrierten aromatischen Verbindungen (Nitroaromaten, Nitrophenolderivaten, Nitrobenzoesäuren etc.) verunreinigt sind.

Nitrierte aromatische Verbindungen gelten in biologischen Kläranlagen als schwer abbaubar. Anorganische Nitrate sind zum Teil für den erhöhten Stickstoffeintrag in die Natur verantwortlich. Die Abwässer einer Produktion von Nitroaromaten müssen deshalb einer aufwendigen Abwasserreinigung unterworfen werden, um diese Schadstoffe aus dem Abwasser zu entfernen, bevor sie an eine Kläranlage abgegeben werden können.

Ein prinzipiell anderer Weg zur Abtrennung der wäßrigen Säure ist die Kristallisation der Nitroaromaten aus der Lösung, In US 5 057 632 wird beschrieben, wie Dinitrotoluol durch einstufige Kristallisation aus der Reaktionslösung abgetrennt werden kann, Damit ist jedoch eine Verschiebung des Isomerenverhältnisses im Dinitrotoluol verbunden, Eine weitere Reinigung des kristallisierten Dinitrotoluols von anhaftenden anorganischen und organischen Säuren wird nicht beschrieben.

Aus DE 2 926 947 geht hervor, daß nach der Nitrierung durch Kristallisation der Nitroaromaten Nitrosierungsmittel aus den Nitroaromaten entfernt werden können. Die auskristallisierende Verbindung muß dann allerdings abgetrennt und neutral gewaschen werden, um weitere organische und anorganische Säuren aus dem Produkt zu entfernen.

Aufgabe war es daher, ein Verfahren zur Verfügung zu stellen, daß es gestattet, reine aromatische Nitroverbindungen herzustellen, ohne daß gleichzeitig technisch aufwendige und kostspielige Reinigungsschritte durchgeführt werden müssen und große Mengen an Abwasser entstehen.

Diese Aufgabe konnte durch das erfindungsgemäße Verfahren gelöst werden.

Gegenstand der Erfindung ist ein Verfahren zur Herstellung von reinen aromatischen Nitroverbindungen, wie sie als Isomerengemisch bei der Nitrierung anfallen, durch Nitrierung der entsprechenden aromatischen Verbindungen mit wäßriger Salpetersäure oder Nitriersäure bei Temperaturen, die oberhalb des Festpunktes der organischen Phase liegen, wobei eine Emulsion aus wäßriger flüssiger Phase in organischer flüssiger Phase oder umgekehrt oder eine Lösung anfällt, und anschließender Trennung der wäßrigen Phase von der organischen Phase bzw. Überführung der Lösung mittels Hilfsstoffen in zwei Phasen mit anschließender Trennung der zwei Phasen oder Destillation der Salpetersäure aus der Lösung, dadurch gekennzeichnet, daß die organische Phase einer oder mehrerer Schmelzkristallisationen unterworfen wird, wobei die nach der oder den Kristallisationen verbleibenden Restschmelzen abgetrennt und in die Nitrierung und/oder in den Reaktionsablauf vor der Phasentrennung zurückgeführt werden.

Durch das erfindungsgemäße Verfahren werden alle anorganischen und organischen Säuren aus den aromatischen Nitroverbindungen entfernt, ohne daß eine wäßrige neutrale oder alkalische Wäsche oder eine Neutralisation der aromatischen Nitroverbindungen notwendig ist. Dadurch fallen auch keine anorganisch oder organisch belasteten Waschwässer an.

Als geeignete Schmelzkristallisation kann die Suspensionskristallisation oder die Schichtkristallisation durchgeführt werden.

Nach Abtrennung der sogenannten Restschmelze werden die Kristalle bei der Schmelzkristallisation durch sogenanntes "Schwitzen", das heißt partielles Aufschmelzen und Abtrennen der flüssigen Phase, von anhaftenden Verunreingungen befreit. Die Restschmelze wird in die Nitrierung zurückgeführt.

Durch das erfindungsgemäße Verfahren werden die sauren Bestandteile in die Nitrierung zurückgeführt und/oder der Säureaufbereitung zugeführt. Außerdem entspricht die Isomerenverteilung des Produktes nach der Schmelzkristallisation der Isomerenverteilung, wie sie bei der Nitrierung anfällt.

Als aromatische Verbindungen, die nitriert werden, seien beispielhaft folgende Verbindungen genannt: 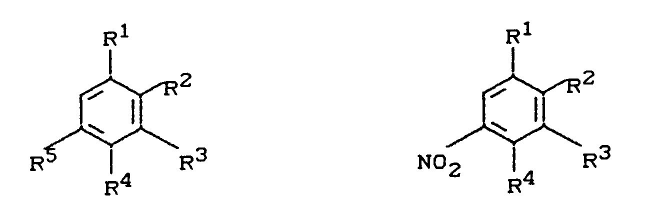
wobei R¹, R², R³, R⁴, R⁵ unabhängig voneinander Wasserstoff, Alkyl, Aryl, F, Cl, Br, J, NO₂, O-Alkyl, O-Aryl, OH bedeuten können.

Bevorzugt nitriert werden Benzol, Nitrobenzol, Toluol, Nitrotoluol, Chlorbenzol, Dichlorbenzol, Chlortoluol, Dinitrotoluol oder deren Isomerengemische.

Die Erfindung soll anhand der nachfolgenden Beispiele näher erläutert werden.

### Beispiel 1

Zu 92,1 g (1,0 Mol) Toluol werden 450,0 g (7,0 Mol) 98 %ige Salpetersäure so zugetropft, daß eine Temperatur von 70°C nicht überschritten wird (Reaktions- und Nachrührphase ca. 1 h). Anschließend werden im Vakuum Säure und Reaktionswasser soweit wie möglich abdestilliert. Das im Sumpf verbleibende nitrierte Produkt wird mit Restschmelze aus Vorversuchen versetzt und auf Raumtemperatur abgekühlt. Der kristallisierte Anteil wird durch Saugfiltration vom flüssigen Anteil getrennt. Das Kristallisat enthält keine Säure mehr. Eine wäßrige Nachbehandlung ist nicht nötig. Das hergestellte Dinitrotoluol hat ein Isomerenverhältnis von 2,4-Dinitrotoluol zu 2,6-Dinitrotoluol von 8 zu 2. Der flüssige Anteil des Nitroproduktes (Restschmelze) wird dem nächsten Kristallisationsansatz zugeschlagen.

### Beispiel 2

584,8 g rohes Dinitrotoluol aus einer Reaktion von Toluol mit Nitriersäure wird mit einer Geschwindigkeit von ca. 0,3°C/Min. von ca. 65°C bis auf 37°C abgekühlt. Anschließend wird der kristalline Anteil durch Absaugen von der Restschmelze getrennt. Die Kristalle enthalten keine Säure oder andere störende Verunreinigungen, so daß eine zusätzliche Reinigung, wie z.B. Wäsche nicht nötig ist.

### Beispiel 3

447,4 g eines Gemisches von rohem Dinitrotoluol (DNT) aus einer Nitrierung und Restschmelze mit folgender Zusammensetzung: 3,35 Gew.-% 2,3-DNT, 56,57 Gew.-% 2,4-DNT, 1,05 Gew.-% 2,5-DNT, 34,88 Gew.-% 2,6-DNT, 4,14 Gew.-% 3,4-DNT wurde mit einer Geschwindigkeit von 0,2°C/Min von ca. 65°C auf 28°C abgekühlt. Die abgesaugten Kristalle hatten folgende Isomerenzusammensetzung: 2,03 Gew.-% 2,3-DNT, 76,25 Gew.-% 2,4-DNT, 0,57 Gew.-% 2,5-DNT, 19,06 Gew.-% 2,6-DNT, 2,10 Gew.-% DNT. Das Isomerenverhältnis von 2,4-DNT zu 2,6-DNT betrug 8 zu 2. Die Kristalle enthalten keine störenden Verunreinigungen und keine Säure mehr, so daß eine zusätzliche Wäsche entfällt.

## Patentansprüche

1. Verfahren zur Herstellung von reinen aromatischen Nitroverbindungen, wie sie als Isomerengemisch bei der Nitrierung anfallen, durch Nitrierung der entsprechenden aromatischen Verbindungen mit wäßriger Salpetersäure oder Nitriersäure bei Temperaturen, die oberhalb des Festpunktes der organischen Phase liegen, wobei eine Emulsion aus wäßriger Phase in organischer flüssiger Phase oder umgekehrt bzw. eine Lösung anfällt, und anschließender Trennung der wäßrigen Phase von der organischen Phase bzw. Überführung der Lösung mittels Hilfsstoffen in zwei Phasen mit anschließender Trennung der zwei Phasen oder Destillation der Salpetersäure aus der Lösung, dadurch gekennzeichnet, daß die organische Phase einer oder mehrerer Schmelzkristallisationen unterworfen wird, wobei die nach der oder den Kristallisationen verbleibenden Restschmelzen abgetrennt und in die Nitrierung oder in den Reaktionsablauf vor der Phasentrennung zurückgeführt werden.

2. Verfahren gemäß Anspruch 1, dadurch gekennzeichnet, daß reine Dinitrotoluolisomerengemische, wie sie bei der Nitrierung von Toluol bzw. von Mononitrotoluolen anfallen, durch Nitrierung von Toluol bzw. Mononitrotoluolen mit wäßriger Salpetersäure oder Nitriersäure hergestellt werden.

## Claims

1. Process for the preparation of pure aromatic nitro compounds, as an isomer mixture such as arises in nitration, by nitration of the corresponding aromatic compounds with aqueous nitric acid or nitrating acid at temperatures above the temperature at which the organic phase is solid, wherein there arises an emulsion of aqueous phase in organic liquid phase, or vice versa, or a solution, and subsequent separation of the aqueous phase from the organic phase or conversion of the solution by means of auxiliary substances into two phases, with subsequent separation of the two phases or distillation of the nitric acid from the solution, characterised in that the organic phase is subjected to one or more melt crystallisations, wherein the residual melts remaining after the crystallisation or crystallisations are separated and returned into the nitration or into the reaction sequence upstream of the phase separation.

2. Process according to Claim 1, characterised in that pure dinitrotoluene isomer mixtures such as occur in nitration of toluene or of mononitrotoluenes are prepared by nitration of toluene or of mononitrotoluenes with aqueous nitric acid or nitrating acid.

## Revendications

1. Procédé de préparation de composés nitrés aromatiques purs, tels qu'ils apparaissent sous forme de mélange d'isomères lors de la nitration, par nitration des composés aromatiques correspondants avec de l'acide nitrique aqueux ou un mélange sulfonitrique à des températures qui sont supérieures au point de solidification de la phase organique, avec formation d'une émulsion constituée par une phase aqueuse dans une phase liquide organique, ou inversement, ou d'une solution, et séparation subséquente de la phase aqueuse d'avec la phase organique ou transformation de la solution en deux phases à l'aide d'adjuvants avec séparation subséquente des deux phases ou distillation de l'acide nitrique à partir de la solution, caractérisé en ce que la phase organique est soumise à une ou plusieurs cristallisations à l'état fondu, les masses fondues résiduelles demeurant après la ou les cristallisations étant séparées et recyclées dans la nitration ou dans le processus réactionnel avant la séparation des phases.

2. Procédé selon la revendication 1, caractérisé en ce que des mélanges purs d'isomères du dinitrotoluène, tels qu'ils apparaissent lors de la nitration du toluène ou de mononitrotoluènes, sont préparés par nitration du toluène ou de mononitrotoluènes avec de l'acide nitrique aqueux ou un mélange sulfonitrique.
